# EUROPEAN PATENT SPECIFICATION

(11) **EP 3 347 081 B1**
(45) Date of publication and mention of the grant of the patent: **04.03.2020**
(21) Application number: 15771668.9
(22) Date of filing: 09.09.2015
(51) Int. Cl.: A61M 37/00

(54) **APPARATUS FOR DELIVERY OF SUBSTANCES INTO BONE**
VORRICHTUNG ZUR FREISETZUNG VON SUBSTANZEN IN KNOCHEN
APPAREIL POUR L'ADMINISTRATION DE SUBSTANCES DANS L'OS

(43) Date of publication of application: 18.07.2018
(73) Proprietor: Revenio Research Oy, 01510 Vantaa (FI)
(72) Inventor: MOILANEN, Petro, 40900 Säynätsalo (FI); NIEMINEN, Heikki, 00170 Helsinki (FI); SALMI, Ari, 00640 Helsinki (FI); HÆGGSTRÖM, Edward, 00140 Helsinki (FI); GARCÍA PÉREZ, Alejandro, 00560 Helsinki (FI)
(74) Representative: Finnpatent Oy
(86) International application number: PCT/FI2015/050589
(87) International publication number: WO 2017/042422

(56) References cited:
- US-A1- 2014 257 144
- US-A1- 2014 303 525
- US-A1- 2015 125 808
- US-A1- 2015 209 566
- US-B1- 6 231 528
- LETFULLIN RENAT R ET AL: "Space simulations of thermal fields generated in bone tissue for application to nanophotohyperthermia and nanophotothermolysis", PHOTONIC THERAPEUTICS AND DIAGNOSTICS VII, SPIE, 1000 20TH ST. BELLINGHAM WA 98225-6705 USA, vol. 7883, no. 1, 10 February 2011 (2011-02-10), pages 1-10, XP060006127, DOI: 10.1117/12.873195 [retrieved on 2011-02-17]
- NIEMINEN H J ET AL: "Ultrasonic transport of particles into articular cartilage and subchondral bone", ULTRASONICS SYMPOSIUM (IUS), 2012 IEEE INTERNATIONAL, IEEE, 7 October 2012 (2012-10-07), pages 1869-1872, XP032434383, ISSN: 1948-5719, DOI: 10.1109/ULTSYM.2012.0469 ISBN: 978-1-4673-4561-3

## Description

### Field of the Invention

The invention relates to bone healthcare and health management. The invention deals with detection of a weak bone and healing of the weak or fractured bone *in vivo.*

Bone diseases are disorders in remodeling of bone tissue. As a result, bones can become mechanically weak. Reduction of bone mineral density (BMD) is a natural process related to aging after the age of 20. However, some bone diseases, such as osteoporosis, can cause excessive loss of BMD. Deficiencies in nutrient intake (e.g., calcium and vitamin D and C), hormonal imbalance and cell abnormalities can also cause bone disorders.

Bone fractures are labelled low-impact fractures and high-impact fractures. The low-impact (or fragility) fractures are predominantly caused by deteriorated bone strength, which results from aging or bone disease, and can occur due to a mechanical impact following e.g. slipping or falling. High-impact (or traumatic) fractures require excessive stress caused by traumatic accidents and can occur in healthy bone. Bone is considered weak when the risk for fragility fractures is increased.

### State of the Art

There is a need for methods to detect and heal weak bones, preferably before fractures occur.

Localized inference of bone quality techniques are being developed by several research groups. To this end, quantitative ultrasound (QUS) is one of the most promising approaches. Yet, ultrasonic detection of clinically relevant fracture sites such as the hip and vertebrae is challenging and requires further development.

Weak bone is typically treated by systemic delivery of drug and growth factors. Such drugs and drug-like factors are absorbed throughout the body. Therefore, high doses may be required to gain sufficient therapeutic effects in the bone. However, the drug, especially at high drug doses, may cause side effects outside fracture sites, some of which may be severe.

Tissue treatment based on localized delivery and release of drugs has been reported for soft tissue sites. In particular, a recent report details ultrasound-aided delivery and release in articular cartilage (Nieminen et al., Ultrasound Med Biol 41(8):2259-2268, 2015) and subchondral bone through articular cartilage (Nieminen et al., Ultrasonics Symposium (IUS), 2012 IEEE International, pages 1869 - 1872). For bone metastases, there are reports on localized ultrasound-aided release of drugs, first transported into the vicinity of the therapy site by blood circulation (Staruch et al., Radiology 263(1):117-127, 2012). However, there is no known method to do simultaneous release and deposition. Moreover, there is no known methodology that would permit construction of a hand-held device for detection of weak bone (site with fracture risk) followed by instant localized treatment.

In prior art document US6231528 B1 is presented an in vivo technology for using ultrasound in conjunction with a biomedical compound or bone growth factor to induce healing, growth and ingrowth responses in bone. To this end, non-invasively applied ultrasonic stimulus is operative to transport the bone growth factor from the external surface of the soft tissue to the bone and to synergistically enhance the interaction between the bone growth factor and the bone. This technology does not involve deposition of the ultrasonically transported objects and describes the use of ultrasound for delivery only in the context of extracorporeal ultrasound transducer, ultrasound pulser, biomedical compounds and bone growth factors. In addition, the technology does not incorporate focused ultrasonic waves which are vital for highly localized treatment.

Furthermore, reference is made to document US2015/125808 A1.

### Short description of the invention

The object of this invention is to accomplish an improved technology for transport and deposition of objects into bone for effective and controllable localized management of bone health. This is achieved by a kit, comprising an object delivery arrangement for delivering objects into bone, and retention means configured to counteract the passive diffusion out from the target and formed by one of a covering layer having a lower perfusion coefficient than the embracing tissues, an active object having a size sufficient to prevent passive diffusion out of the target in the bone, a substance that expands and covers the target site in the bone, subsequent application of mechanical waves for maintaining the substance in the target, after application of mechanical waves for depositing the substance, said arrangement comprising an ultrasound, photo-acoustics or plasma source configured to generate localized mechanical waves, and said arrangement being configured to perform localized deposition of the objects near bone, expose the objects and the bone to said localized mechanical waves to force the objects into the bone, and perform retention of the deposited objects in the bone, by using said retention means, so as to prevent the deposed objects from escaping the target site in the bone.

The invention is defined in the appended set of claims. The invention is based on generation of localized mechanical waves into a tissue, and localized deposition of the objects near bone, and deposition of the objects to the bone by the effect of said mechanical waves.

The direction of transportation and deposition of objects into bone tissue is not limited to transport and deposition from bone periosteal (i.e. outer) surface into bone tissue. The transportation and deposition of objects can also be achieved from any surface of a cavity (e.g. endosteal surface) or pore into bone tissue.

The benefit of the invention is that the proposed conjunction of means permits an enhanced therapeutic power and advanced management of the therapeutic effect compared to the closest prior art.

### Short description of figures

- Figure 1: shows preferred embodiments of the invention.
- Figure 2: shows alternative embodiments of the invention.
- Figure 3: shows preliminary results.

### Detailed description of the invention

In the present invention is presented an object delivery arrangement for delivering objects into bone. The delivery object is, for example, a drug molecule or molecules for osteoporosis treatment. The arrangement comprises means for generating localized mechanical waves into a tissue. Said means are for example at least one of mechanical wave emitter 108, 113, energy conductor 109, sound source 111, and waveguide 112. Said means 108, 111 are for example an ultrasound transducer or an ultrasound source. The arrangement further comprises means for performing localized deposition of the objects 103 contained within a material boundary 104 near bone interface 107, and means for exposing the objects and the bone to said mechanical waves, obtaining deposition 110 of the objects to the bone. Said means for performing localized deposition are for example at least one of hollow structure 101, 201, cutting edge 102, reservoir 200 and syringe 203. The means for exposing are for example at least one of means according to reference signs 108, 109, 111, 112 and 113. The means 108, 109, 111, 112, 113 can penetrate skin 105 or tissues 106.

The arrangement according to the present invention preferably comprises means for transporting the objects to the bone 107 in order to obtain deposition of the objects 110 to the bone. The means for transporting are for example at least one of means according to reference signs 101, 102, 103, 108 and 109. In one embodiment the location of weak bone is detected by quantitative ultrasound (QUS) or ultrasound imaging. After defining the weak bone, the deposition of objects into the weak bone can be achieved with mechanical waves generated with the same or a different ultrasound system than used for QUS or ultrasound imaging.

The arrangement according to the present invention can comprise means, i.e. at least one of means according to reference signs 108, 113, 109, 111, 112 and 204a-e, for generating localized mechanical waves into the tissue to perform the localization on the basis of at least one of high-intensity focused ultrasound (HIFU) 204c, topological guides for ultrasound 112 and electromagnetic steering of the objects to improve focusing of the diffusion. It is essential for localized deposition to localize the driving mechanical (or sound) wave field inside the tissue, at the preferred point at or near the bone (e.g. a weak part of the bone). Localization of the driving mechanical wave field is realized either by means of high-intensity focused ultrasound (HIFU) or topological ultrasound (waveguides or high-order topologies i.e. fractal structures). Localization can also be realized by means of a counter electrode, or electromagnetic fields that steer the field or objects, to improve focusing of the diffusion.

In one embodiment according to the present invention the arrangement can comprise means, i.e. at least one of means according to reference signs 108, 113, 109, 111, 112 and 204a-e, for generating localized mechanical waves into a tissue on the basis of time reversal ultrasound performing adaptive focusing. Time reversal ultrasound permits adaptive focusing through an inhomogeneous medium, such as soft tissue or trabecular bone.

The arrangement can also comprise means, i.e. at least one of means according to reference signs 108, 113, 109, 111, 112, 200, 201, 203 and 204a-e, for performing localized deposition of the objects near bone based on photo-acoustic transformation in order to generate mechanical waves near objects. Photo-acoustic transformation also permits introduction of the sound source inside the tissue. In this approach the tissue is irradiated by electromagnetic waves (e.g. laser pulse), which penetrate and absorb into the tissue. The absorption causes localized thermal expansion, which results in emission of mechanical waves (i.e. sound field) at the point of thermal expansion. The resulting sound field is tunable by parameters of the optical beam (e.g. wavelength, pulse duration and geometric size and shape of the optical beam, number of illuminated spots and temporal phasing of the onset of the illumination of the different spots). These parameters affect the penetration depth, absorption and scattering in the tissue and determine the emitted sound field. The energy of the electromagnetic beam can be absorbed in any parts of the tissue or in the objects that are being deposited or a combination of thereof. In particular, the optical absorption coefficients characteristic to different layers of the soft tissue and bone are functions of the optical wavelength. Thereby, tuning of the optical wavelength permits e.g. maximization of the absorption ratio between the bone and soft tissue and results localization of the sound source at or near the bone. The localization can be also obtained by using a point source (e.g. 108, 113, 204a-b), independent of the technique of implementation.

In another embodiment according to the present invention, the arrangement comprises means for selecting the objects from a reservoir of objects and forcing the objects into the bone. Objects (e.g. molecules) are selected from the reservoir of objects (e.g. solution) and are forced into the bone.

The arrangement can comprise means, i.e. at least one of means according to reference signs 108, 113, 109, 111, 112, 200, 201, 203 and 204a-e, for performing retention of objects 103, 110 by depositing in addition to the objects a covering layer having a lower perfusion coefficient than the embracing tissues. The purpose of retention is to prevent the deposed objects from escaping the target (bone). Retention is realized by deposing another covering layer that has much lower perfusion coefficient compared to those of the embracing tissues. This can also be realized by depositing a substance that expands and covers the target. An alternative embodiment to this approach is using an active object, which is too large for passive diffusion, but can be actively deposited using the method described in the invention. The large size then prevents passive diffusion out of the target. Retention can also be controlled by subsequent sonication: the first application of mechanical waves deposits the substance into the target, followed by several applications of mechanical waves that maintain the substance in the target (counteract the passive diffusion out from the target).

In one further embodiment according to the present invention the arrangement can comprise means, i.e. at least one of means according to reference signs 101, 102, 103, 108, 113, 109, 111, 112, 200, 201, 203 and 204a-e, for activating objects selectively at different time points. Objects that are inactive in the tissue are first driven in, to form a reservoir 103, 104, 110 of the objects in the tissue. After this, the objects are collectively or selectively activated e.g. by means of mechanical waves, electromagnetic waves or temperature. Selective activation permits activation at different time points, e.g. one ingredient of the objects can be activated directly after drive in and another ingredient can be activated later. This can be considered to be e.g. catalyzation. In an alternative embodiment, the different drugs are encapsulated in or on surface of e.g. gas voids (with or without lipid shells or equivalent) of various sizes corresponding to various resonant frequencies. After driving in, the encapsulated objects are released at desired moments by sonicating at the resonant frequency corresponding to the release of objects desired.

The arrangement can also comprise means, i.e. at least one of means according to reference signs 108, 113, 109, 111, 112, 204a-e, for affecting tissue 107, 105, 106, 110 and 205 by mechanical vibrations. In assembly in situ or in vivo embodiments one, two or several components are driven in the tissue and then treated (shaken) by mechanical vibration. This shaking causes merging of the components to larger aggregates that cannot escape from the target tissue (e.g. bone) or whose escape rate is decreased.

In nanotechnology embodiments according to the present invention the arrangement according to the present invention can comprise nanostructure means to control diffusion and to amplify the diffusion. Nano-swimmers or functionalized nano-rods permit improved control of the diffusion and amplification of the diffusion. The same can also be accomplished e.g. by nano motors, which are controlled by at least one of external field, internal field, external power source and internal power source.

In one further embodiment according to the present invention the arrangement can comprise means (204a-e) for exposing the objects and the bone to said mechanical waves to deposit the objects to the bone utilizing at least one of blood circulation and the bone marrow cavity for the transportation of the objects. Alternatively, instead of driving from the periosteal side of the bone, the objects are driven in bone from the inside (e.g. endosteal side) (means 204b), utilizing blood circulation and/or the bone marrow cavity for the initial transport of the objects to the treatment site, e.g. the fracture site, and then exploiting mechanical waves to deposit the objects into the bone.

In further embodiments the arrangement according to the present invention can comprise multi-center-frequency means, i.e. at least one of means according to reference signs 108, 113, 109, 111, 112 and 204a-e, to generate mechanical waves of at least two different frequencies in order to improve transportation of the objects and deposition of the objects.

Generation of sound waves by at least at two distinct center-frequencies can enhance the drive in. For instance, a kilohertz frequency transducer (e.g. 204d) can be used to increase the permeability at the bone surface (e.g. periosteum or endosteum) and a megahertz frequency transducer (e.g. 204e) can be used to push the objects in.

In embodiments according to the present invention the means, i.e. at least one of means according to reference signs 108, 113, 109, 111, 112 and 204a-e, for generating localized mechanical waves into a tissue can comprise a plasma source. Alternatively, instead of using a conventional ultrasound or photo-acoustic approach, the driving pressure field is generated by a plasma source. The plasma source can be realized e.g. by a focused laser or a spark gap.

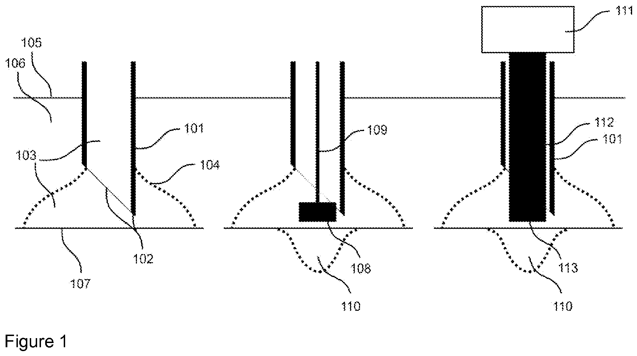

Figure 1 depicts the preferred embodiment of the invention. A catheter (101) featuring a cutting edge (102) perforates the skin and tissue. The catheter delivers the objects (103) and it forms an object reservoir (103) with boundary 104 near the bone surface (107). The sound source comprises energy conductor (109) and a sound emitter (108). The sound emitter 108 may be e.g. a flat piezo, a focused piezo, spark, laser induced spark, EMUT (Energy Mode Ultrasound Transducer), CMUT (Capacitive micromachined ultrasonic transducers), PMUT (Piezoelectric Micromachined Ultrasonic Transducers) and equivalent. The mechanical wave generated by the sound emitter translates the objects into the bone (110). In another embodiment the sound source (111) is located outside the tissue and the mechanical wave is transmitted to the tissue and active ingredient via a waveguide (112). In one embodiment of the invention e.g. 10 - 500kHz mechanical wave is transmitted through at least one of waveguide (112), active ingredient (103), tissue (106) and sound emitter (108). This mechanical wave alters the permeability of the bone membrane. Another, e.g. 0,5 - 50 MHz mechanical wave is subsequently transmitted to the boundary. This mechanical wave deposits the active ingredient from the reservoir to the bone.

According to one embodiment 111 is a light source and the light wave is guided to reservoir 103, 104 and bone 107 through an optical fiber 112 or reflecting inner wall of a catheter 101. The light wave is absorbed by active ingredient 103 or bone 107 to generate light-induced sound waves for translating the active ingredient 103 into the bone 107.

The object can be e.g. molecules, drugs, vehicles carrying the object, imaging contrast agent, minerals or nanofibers. Biologically active materials that may be of interest include analgesics, antagonists, anti-inflammatory agents, anthelmintics, antianginal agents, antiarrhythmic agents, antibiotics (including penicillins), anticholesterols, anticoagulants, anticonvulsants, antidepressants, antidiabetic agents, antiepileptics, antigonadotropins, antihistamines, antihypertensive agents, antimuscarinic agents, antimycobacterial agents, antineoplastic agents, antipsychotic agents, immunosuppressants, antithyroid agents, antiviral agents, antifungal agents, anxiolytic sedatives (hypnotics and neuroleptics), astringents, beta-adrenoceptor blocking agents, blood products and substitutes, anti-cancer agents, cardiacinotropic agents, contrast media, corticosterioids, cough suppressants (expectorants and muco-lytics), diuretics, dopaminergics (antiparkinsonian agents), haemostatics, immunosuppressive and immunoactive agents, lipid regulating agents, muscle relaxants, parasympathomimetics, parathyroid calcitonin and biphospho-nates, prostaglandins, radiopharmaceuticals, sex hormones (including steroids), anti-allergic agents, stimulants and anorexics, sympathomimetics, thyroid agents, vasidilators, neuron blocking agents, anticholinergic and choli-nomimetic agents, antimuscarinic and muscarinic agents, vitamins, and xanthines. Exemplary medicaments can be e.g. ibandronic acid, zolendronic acid, teriparatide, denosumab, TGF-beta, FGF-beta and BB1/biopharm, According to one embodiment, the sound emitter (108) is a confocal transducer featuring two transducers of different center frequencies. According to one embodiment, the two frequencies generate a third frequency which acts as the wave translating the active ingredient.

According to one embodiment, catheter wall (101) or waveguide (112) acts as a "cold finger", i.e. heat energy is absorbed from the tissues exposed to ultrasound induced heating.

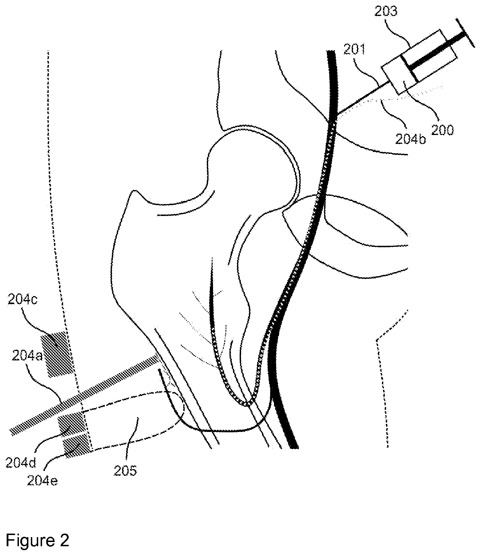

Figure 2 depicts another means of translating objects, e.g. active ingredients, into bone. A syringe (203), loaded with the objects containing active ingredient (200), is connected with a needle (201) to a major artery which transports the objects with the blood flow to the treatment site. An ultrasound generator (204a), comprising at least one of 101, 103, 108, 111, 112, 113, 109 generates the ultrasound which locally translates the drug into the bone. In an alternative embodiment the ultrasound system operates intravenously (204b). In another alternative embodiment, the ultrasound waves are focused through the skin and tissue to the bone - reservoir 103 boundary 104, 107 with an ultrasound generator (204c). In another alternative embodiment, a combination ultrasound system comprising of two different transducers, one of which (204d) translates the active ingredient 205 through the tissue and skin (such as sonophoresis), whereas the other one (204e) translates the active ingredient into the bone.

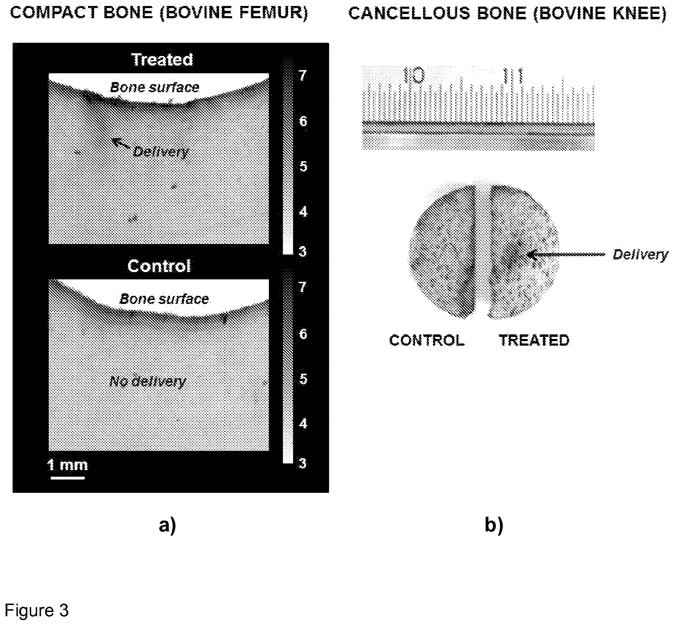

Figure 3 shows preliminary results on compact cortical and spongy cancellous bone. (a) Optical microscopy image of cortical bone into which we have delivered contrast agent (methylene blue; image on top) by using high-intensity focused ultrasound (HIU) (Parameters: sine burst frequency: 2.17 MHz; cycles per burst: 200, pulse-repetition frequency: 1000 Hz). The gray scale represents optical absorption. The ultrasound beam enhanced the delivery, as is indicated by an arrow. There is no similar effect seen in a control sample (image on bottom), extracted from the same piece of bone and treated consistently but without ultrasound. (b) Photograph of the result of a related experiment in cancellous bone (sine burst frequency: 2.17 MHz; cycles per burst: 100, pulse-repetition frequency: 600 Hz).

Localized delivery of objects into the bone includes transport and deposition according to the preferred or alternative embodiments of the invention as described in Figures 1 and 2. In one phase, one object or a group of objects is transported into the bone. In the second phase, a second object is transported into bone. The second object is delivered close to the pathways through which the first object has travelled to prevent washout of first object. The second object can alternatively self-assemble with itself or with the first object to create large-sized constructs (e.g. via mechanisms such as self-assembly) to slow down or prevent washout of the objects with therapeutic effect. The role of the second object can also be to catalyze the therapeutic effect of first object. The catalyzation can be achieved also by exposing at least one of the objects to mechanical or electromagnetic waves.

Localized deposition is realized by employing one of the presented or different combinations of the presented techniques and methods.

## Claims

1. A kit, comprising
an object delivery arrangement (101, 111, 201, 203, 204a) for delivering objects (110) into bone, and retention means (108, 113, 109, 111, 112, 200, 201, 203, 204a-e) formed by one of
- a covering layer having a lower perfusion coefficient than the embracing tissues,
- an active object having a size sufficient to prevent passive diffusion out of the target in the bone,
- a substance configured to expand and cover the target site in the bone, and
- means for subsequent application of mechanical waves for maintaining the substance in the target, after application of mechanical waves for depositing the substance,
wherein said object delivery arrangement comprises an ultrasound, photo-acoustics or plasma source configured to generate localized mechanical waves, and
wherein said object delivery arrangement is configured to
- perform localized deposition of the objects near bone,
- expose the objects and the bone to said localized mechanical waves to force the objects into the bone, and
- wherein said retention means is configured to perform retention of the deposited objects in the bone, so as to prevent the deposed objects from escaping the target site in the bone.

2. A kit according to claim 1, **characterised in that** the object delivery arrangement comprises means (101, 102, 103, 108, 109) for transporting the objects to the bone in order to obtain deposition of the objects into the bone.

3. A kit according to claim 1, **characterised in that** the object delivery arrangement comprises means (108, 113) for generating localized mechanical waves into the tissue performing the localization on the basis of at least one of high-intensity focused ultrasound (HIFU), waveguide, electromagnetic steering of the wave field and electromagnetic steering of the object in order to improve focusing of the diffusion.

4. A kit according to claim 1, **characterised in that** the object delivery arrangement comprises means (108, 113) for generating localized mechanical waves into a tissue on the basis of time reversal ultrasound performing adaptive focusing.

5. A kit according to claim 1, **characterised in that** the object delivery arrangement comprises means for performing localized deposition of the objects near bone on the basis of photo-acoustic transformation in order to localize the means for generating localized mechanical waves inside the tissue and in order to reduce ultrasonic energy deposition in tissue adjacent to bone relative to that in bone.

6. A kit according to claim 1, **characterised in that** the object delivery arrangement comprises means for selecting objects from the reservoir of objects and forcing the objects into the bone tissue.

7. A kit according to claim 1, **characterised in that** the object delivery arrangement comprises means for activating objects selectively at different time points.

8. A kit according to claim 1, **characterised in that** the object delivery arrangement comprises means for affecting a target structure by mechanical vibrations for enhanced deposition.

9. A kit according to claim 1, **characterised in that** the object delivery arrangement comprises nanostructure means to achieve at least one of control diffusion and amplification of the diffusion.

10. A kit according to claim 1, **characterised in that** the object delivery arrangement comprises means (204a-e) for exposing the objects and the bone to said mechanical waves to obtain deposition of the objects to the bone utilizing at least one of blood circulation and the bone marrow cavity for the transportation of the objects.

11. A kit according to claim 1, **characterised in that** the object delivery arrangement comprises multi-center-frequency means to generate mechanical waves of at least two different frequencies in order to improve transportation of the objects and deposition of the objects.

## Patentansprüche

1. Kit, umfassend
eine Anordnung (101, 111, 201, 203, 204a) zur Freisetzung von Objekten (110) in Knochen, und
ein Rückhaltemittel (108, 113, 109, 111, 112, 200, 201, 203, 204a-e), gebildet aus einem von
- einer Abdeckschicht mit einem niedrigeren Perfusionskoeffizienten als die umgebenden Gewebe,
- ein aktives Objekt mit einer Größe, die dafür ausreichend ist, eine passive Diffusion aus dem Zielbereich in dem Knochen zu verhindern,
- eine Substanz, die dazu konfiguriert ist, sich auszudehnen und den Zielbereich in dem Knochen abzudecken, oder
- ein Mittel zum darauffolgenden Aufbringen von mechanischen Wellen, um die Substanz nach dem Aufbringen von mechanischen Wellen zum Ablagern der Substanz in dem Zielbereich zu halten,
wobei die Anordnung zur Freisetzung von Objekten eine Ultraschall-, Photoakustik- oder Plasmaquelle umfasst, die dazu konfiguriert ist, lokalisierte mechanische Wellen zu erzeugen, und
wobei die Anordnung zur Freisetzung von Objekten dazu konfiguriert ist,
- eine lokalisierte Ablagerung der Objekte nahe Knochen auszuführen,
- die Objekte und den Knochen den lokalisierten mechanischen Wellen auszusetzen, um die Objekte in den Knochen zu treiben, und
wobei das Rückhaltemittel dazu konfiguriert ist, eine Rückhaltung der abgelagerten Objekte in dem Knochen zu bewirken, um so zu verhindern, dass die abgelagerten Objekte den Zielbereich in dem Knochen verlassen.

2. Kit nach Anspruch 1, **dadurch gekennzeichnet, dass** die Anordnung zur Freisetzung von Objekten ein Mittel (101, 102, 103, 108, 109) zum Transportieren der Objekte zu dem Knochen umfasst, um eine Ablagerung der Objekte in dem Knochen zu bewirken.

3. Kit nach Anspruch 1, **dadurch gekennzeichnet, dass** die Anordnung zur Freisetzung von Objekten ein Mittel (108, 113) zum Erzeugen von lokalisierten mechanischen Wellen in dem Gewebe umfasst, wobei die Lokalisierung auf Basis von hochintensiver fokussierter Ultraschall (HIFU)-, Wellenleiter-, elektromagnetischer Steuerung des Wellenfelds und/oder elektromagnetischer Steuerung des Objekts ausgeführt wird, um die Fokussierung der Diffusion zu verbessern.

4. Kit nach Anspruch 1, **dadurch gekennzeichnet, dass** die Anordnung zur Freisetzung von Objekten ein Mittel (108, 113) zum Erzeugen von lokalisierten mechanischen Wellen in einem Gewebe auf Basis von Zeitumkehr-Adaptiv-Fokussierung umfasst.

5. Kit nach Anspruch 1, **dadurch gekennzeichnet, dass** die Anordnung zur Freisetzung von Objekten ein Mittel zum Ausführen einer lokalisierten Ablagerung der Objekte nahe Knochen auf Basis einer photoakustischen Transformation umfasst, um das Mittel zum Erzeugen von lokalisierten mechanischen Wellen in dem Gewebe zu lokalisieren und um die Ablagerung durch Ultraschallenergie in Gewebe benachbart zu Knochen relativ zu der in Knochen zu reduzieren.

6. Kit nach Anspruch 1, **dadurch gekennzeichnet, dass** die Anordnung zur Freisetzung von Objekten ein Mittel zum Auswählen von Objekten aus dem Reservoir von Objekten und zum Treiben der Objekte in das Knochengewebe umfasst.

7. Kit nach Anspruch 1, **dadurch gekennzeichnet, dass** die Anordnung zur Freisetzung von Objekten ein Mittel zum selektiven Aktivieren der Objekte an verschiedenen Zeitpunkten umfasst.

8. Kit nach Anspruch 1, **dadurch gekennzeichnet, dass** die Anordnung zur Freisetzung von Objekten ein Mittel zum Beeinflussen einer Zielbereichsstruktur durch mechanische Vibrationen zwecks verbesserter Ablagerung umfasst.

9. Kit nach Anspruch 1, **dadurch gekennzeichnet, dass** die Anordnung zur Freisetzung von Objekten ein Nanostruktur-Mittel umfasst, um mindestens eine Kontrolldiffusion und/oder eine Verstärkung der Diffusion zu erzielen.

10. Kit nach Anspruch 1, **dadurch gekennzeichnet, dass** die Anordnung zur Freisetzung von Objekten ein Mittel (204a-e) zur Beaufschlagung der Objekte und der Knochen mit den mechanischen Wellen umfasst, um eine Ablagerung der Objekte am Knochen unter Verwendung der Blutzirkulation und/oder der Knochenmarkhöhle für den Transport der Objekte zu erzielen.

11. Kit nach Anspruch 1, **dadurch gekennzeichnet, dass** die Anordnung zur Freisetzung von Objekten ein Multizentrenfrequenz-Mittel umfasst, um mechanische Wellen mit mindestens zwei verschiedenen Frequenzen zu erzeugen, um den Transport der Objekte und die Ablagerung der Objekte zu verbessern.

## Revendications

1. Kit, comprenant
un agencement d'administration d'un objet (101, 111, 201, 203, 204a) pour administrer des objets (110) dans l'os, et un moyen de rétention (108, 113, 109, 111, 112, 200, 201, 203, 204a-e) formé par l'un(e)
- d'une couche de couverture ayant un coefficient de perfusion inférieur aux tissus environnants,
- d'un objet actif ayant une taille suffisante pour empêcher une diffusion passive hors de la cible dans l'os,
- d'une substance configurée pour s'étendre et couvrir le site cible dans l'os, et
- d'un moyen pour l'application consécutive d'ondes mécaniques pour maintenir la substance dans la cible, après l'application d'ondes mécaniques pour déposer la substance,
dans lequel ledit agencement d'administration d'un objet comprend une source d'ultrasons, photo-acoustique ou plasma configurée pour générer des ondes mécaniques localisées, et
dans lequel ledit agencement d'administration d'un objet est configuré pour
- exécuter le dépôt localisé des objets près de l'os,
- exposer les objets et l'os auxdites ondes mécaniques localisées pour forcer les objets dans l'os, et
dans lequel ledit moyen de rétention est configuré pour exécuter la rétention des objets déposés dans l'os de façon à empêcher les objets déposés de s'échapper du site cible dans l'os.

2. Kit selon la revendication 1, **caractérisé en ce que** l'agencement d'administration d'un objet comprend un moyen (101, 102, 103, 108, 109) pour transporter les objets vers l'os afin d'obtenir le dépôt des objets dans l'os.

3. Kit selon la revendication 1, **caractérisé en ce que** l'agencement d'administration d'un objet comprend un moyen (108, 113) pour générer des ondes mécaniques localisées jusque dans le tissu effectuant la localisation sur la base d'au moins un guidage du champ d'ondes par ultrasons focalisés de haute intensité (HIFU), par guide d'ondes, électromagnétique et guidage électromagnétique de l'objet afin d'améliorer la focalisation de la diffusion.

4. Kit selon la revendication 1, **caractérisé en ce que** l'agencement d'administration d'un objet comprend un moyen (108, 113) pour générer des ondes mécaniques localisées à l'intérieur d'un tissu sur la base d'une focalisation adaptative effectuant des ultra-sons à retournement temporel.

5. Kit selon la revendication 1, **caractérisé en ce que** l'agencement d'administration d'un objet comprend un moyen pour exécuter le dépôt localisé des objets près de l'os sur la base de la transformation photo-acoustique afin de localiser le moyen de génération d'ondes mécaniques localisées à l'intérieur du tissu et afin de réduire le dépôt d'énergie aux ultra-sons dans le tissu adjacent à l'os par rapport à celui dans l'os.

6. Kit selon la revendication 1, **caractérisé en ce que** l'agencement d'administration d'un objet comprend un moyen pour sélectionner des objets à partir du réservoir d'objets et forcer les objets à l'intérieur du tissu osseux.

7. Kit selon la revendication 1, **caractérisé en ce que** l'agencement d'administration d'un objet comprend un moyen pour activer des objets de manière sélective à différents points temporels.

8. Kit selon la revendication 1, **caractérisé en ce que** l'agencement d'administration d'un objet comprend un moyen pour affecter une structure cible par des vibrations mécaniques pour un dépôt amélioré.

9. Kit selon la revendication 1, **caractérisé en ce que** l'agencement d'administration d'un objet comprend un moyen de nanostructures pour obtenir au moins une d'une diffusion de contrôle et d'amplification de la diffusion.

10. Kit selon la revendication 1, **caractérisé en ce que** l'agencement d'administration d'un objet comprend un moyen (204a-e) pour exposer les objets et l'os auxdites ondes mécaniques pour obtenir le dépôt des objets sur l'os en utilisant au moins une de la circulation sanguine et de la cavité médullaire pour le transport des objets.

11. Kit selon la revendication 1, **caractérisé en ce que** l'agencement d'administration d'un objet comprend un moyen de fréquences multicentriques pour générer des ondes mécaniques d'au moins deux fréquences différentes afin d'améliorer le transport des objets et le dépôt des objets.
